# EUROPÄISCHE PATENTSCHRIFT

(11) **EP 1 865 998 B1**
(45) Veröffentlichungstag und Bekanntmachung des Hinweises auf die Patenterteilung: **11.07.2012**
(21) Anmeldenummer: 06724082.0
(22) Anmeldetag: 06.04.2006
(51) Int. Cl.: A61L 2/18, A61B 19/00, C11D 3/04, C11D 3/06, C11D 3/30, B08B 3/08

(54) **MASCHINELLE DESINFEKTION VON GEGENSTÄNDEN**
MECHANIZED DISINFECTION OF ARTICLES
DÉSINFECTION D'OBJETS AU MOYEN D'UNE MACHINE

(30) Priorität: 06.04.2005 EP 05007557
(43) Veröffentlichungstag der Anmeldung: 19.12.2007
(73) Patentinhaber: Chemische Fabrik Dr. Weigert GmbH & Co. KG, 20539 Hamburg (DE)
(72) Erfinder: STAFFELDT, Jürgen, 21423 Winsen (DE); WAGEMANN, Wolfgang, 22967 Tremsbüttel (DE); SCHMIDT, Verona, 22605 Hamburg (DE)
(74) Vertreter: Glawe, Delfs, Moll
(86) Internationale Anmeldenummer: PCT/EP2006/003137
(87) Internationale Veröffentlichungsnummer: WO 2006/105967

(56) Entgegenhaltungen:
- WO-A-03/064580
- DE-A1- 10 147 186
- DE-A1- 19 710 255
- US-A- 4 308 229
- US-A- 5 393 789
- US-B1- 6 565 804

## Beschreibung

Die Erfindung betrifft das Gebiet der maschinellen Reinigung und Desinfektion von Gegenständen wie bspw. medizinische und/oder chirurgische Instrumente und Apparate oder Geschirr.

Medizinische und chirurgische Instrumente und Apparate müssen nach dem Gebrauch gereinigt und desinfiziert werden. Dies kann maschinell erfolgen. Die Desinfektion wird in der Regel getrennt von der Reinigung durchgeführt und beinhaltet entweder die Verwendung eines speziellen Mittels (chemische oder chemothermische Desinfektion), oder eine thermische Desinfektion. Auch bei der maschinellen Reinigung von Geschirr (bspw. Beim Spülen von Trinkgläsern) sind Hygienestandards zu erfüllen, die einen Desinfektionsschritt erforderlich machen können.

Die Schrift WO 03/064 580 A1 offenbart die Verwendung eines Reinigungsmittels, das wenigstens zwei verschiedene Tenside enthält, ausgewählt aus wenigstens zwei der drei Gruppen kationische, nichtionische und amphotere Tenside und das anwendungsfertig verdünnt in wässriger Lösung einen pH-Wert von wenigstens 10,5 aufweist, zur Destabilisierung von Prionen bei der maschinellen Reinigung und/oder Desinfektion von medizinischen und/oder chirurgischen Instrumenten und Apparaten.

Der Erfindung liegt die Aufgabe zugrunde, eine Möglichkeit zur Desinfektion von Gegenständen wie medizinischen und/oder chirurgischen Instrumenten und Apparaten zu schaffen, bei der Bakterien, Viren und Pilze (Hefen und Schimmelpilze), insbesondere Bakterien und Pilze, auf wirtschaftlicher Art und Weise abgetötet bzw. inaktiviert werden. Die Erfindung soll zur routinemäßigen Anwendung bei der maschinellen Reinigung und Desinfektion geeignet sein.

Gegenstand der Erfindung ist daher die Verwendung eines Reinigungsmittels gemäß Anspruch 1

Zunächst seien einige im Rahmen der Erfindung verwendete Begriffe erläutert.

Der Begriff Reinigungsmittel bezeichnet jede anwendungsfertige Formulierung, die entweder unmittelbar oder verdünnt mit Wasser zur Reinigung oder Desinfektion der entsprechenden Instrumente Verwendung findet. Im Kontext der Erfindung schließt der Begriff Reinigungsmittel den Begriff Desinfektionsmittel ein. Das Reinigungsmittel kann in fester Form oder vorzugsweise flüssig formuliert sein. Als Reinigungslösung, d.h. anwendungsfertig verdünnt in wässriger Lösung, weist das Reinigungsmittel einen pH-Wert von 10,5 oder grö-βer auf.

Das erfindungsgemäß verwendete Reinigungsmittel enthält wenigstens zwei verschiedene Tenside aus den genannten Tensidgruppen. Damit werden Verbindungen bezeichnet, welche die Grenzflächenspannung herabsetzen, also amphiphile Verbindungen mit mindestens einem hydrophoben und einem hydrophilen Molekülteil. Im Rahmen der Erfindung sind sämtliche Tenside wie beispielsweise anionische Tenside (zusätzlich zu den in Anspruch 1 genannten Tensidgruppen), nichtionische Tenside, kationische Tenside, amphotere Tenside und Blockcopolymere (insbesondere aus Ethylenoxid- und Propylenoxideinheiten) verwendbar. Beispielhaft verwiesen wird auf Römpp Chemielexikon, 10. Aufl., Stichwort "Tenside".

Die Erfindung findet Einsatz bei der maschinellen Reinigung und/oder Desinfektion der Gegenstände wie bspw. medizinischen und oder chirurgischen Instrumenten und/oder Apparaten. "Maschinell" bedeutet, dass das Verfahren vorzugsweise in einer Spülmaschine automatisch abläuft und im Zuge der Reinigung bzw. der Desinfektion kein menschlicher Eingriff erforderlich ist. Insbesondere kann erfindungsgemäß in einer üblichen Spül- und Aufbereitungsmaschine für chirurgische Instrumente oder Geschirr gearbeitet werden. Sie kann insbesondere zur routinemäßigen, täglichen Instrumentenreinigung bzw. Geschirrreinigung verwendet werden.

Die Begriffe "Reinigung und/oder Desinfektion" beinhalten die erforderlichen Schritte bei der Aufarbeitung benutzter Gegenstände bis hin zu einem sauberen Zustand, an den sich vor der Wiederverwendung noch eine Sterilisierung anschlie-βen kann.

Medizinische und/oder chirurgische Instrumente und Apparate sind sämtliche im medizinischen und Krankenhausbereich eingesetzten Geräte sowie Teile davon, die einer maschinellen Reinigung und Desinfektion grundsätzlich zugänglich sind.

Der Begriff Geschirr bezeichnet sämtliche Gegenstände insbesondere aus Glas, Porzellan, Metall oder Kunststoff, die im Küchenbereich zur Zubereitung und/oder Verzehr von Lebensmitteln verwendet werden. Die Erfindung kann bspw. Verwendung finden bei der maschinellen Reinigung und Desinfektion von Trinkgläsern. Insbesondere bei der maschinellen Reinigung von Trinkgläsern in der Gastronomie ist es erforderlich, dass diese nach sehr kurzen Spülzeiten (90 Sek. bis 5 Min.) wieder für eine Weiterverwendung zur Verfügung stehen. Die Erfindung ermöglicht hier eine hinreichende Desinfektion der Gläser auch ohne die im Stand der Technik übliche Einwirkung von Hitze. Dies ist besonders vorteilhaft, da so einerseits der Spülvorgang beschleunigt wird und andererseits die Gläser nicht mit für den sofortigen Ausschank kalter Getränke ungeeignet hohen Temperaturen aus der Spülmaschine entnommen werden.

Abtötung/Inaktivierung von Mikroorganismen bedeutet, dass diese hinreichend abgetötet werden, um eine gefahrlose anschließende bestimmungsgemäße Verwendung der chirurgischen Instrumente und Apparate oder des Geschirrs zu ermöglichen. Anforderungen an chemische Desinfektionsmittel zur Prüfung der bakteriziden Wirkung sind bspw. in DIN EN 13727 und zur Prüfung der fungiziden Wirkung bspw. in DIN EN 13624 niedergelegt. Die viruzide Wirkung wird bspw. gemäß der Richtlinie des Bundesgesundheitsamtes und der Deutschen Vereinigung zur Bekämpfung von Viruskrankheiten e. V. geprüft.

Die Erfindung beruht auf der überraschenden Erkenntnis, dass eine Kombination von Alkalität und wenigstens zwei verschiedenen Tensiden aus der Gruppe bestehend aus kationischen, nichtionischen und amphoteren Tensiden eine hinreichende Wirkung gegen Bakterien, Viren und Pilze bei der maschinellen Instrumentenreinigung und -aufbereitung bzw. beim Geschirrspülen aufweist.

Wenn im Rahmen der Erfindung von wenigstens zwei verschiedenen Tensiden die Rede ist, ist damit gemeint, dass diese aus zwei verschiedenen Gruppen (kationisch, nichtionisch oder amphoter) ausgewählt sind. Wenigstens zwei der genannten drei Gruppen müssen mithin kombiniert werden, damit die erfindungsgemäße Abtötung bzw. Inaktivierung von Mikroorganismen eintritt. Bevorzugt ist eine Kombination von wenigstens drei Tensiden aus allen drei genannten Gruppen.

Es ist erfindungsgemäß insbesondere möglich, sowohl die Reinigung als auch eine Desinfektion unter Verwendung eines einzigen Mittels durchzuführen, so dass die aufwendige und teure Vorratshaltung sowie Dosierung eines separaten Desinfektionsmittels unterbleiben kann. Ferner kann auf die energie- und zeitaufwändige sowie insbesondere für empfindliche Kunststoff- oder Gummiteile belastende Thermodesinfektion bei hohen Temperaturen verzichtet werden.

Der pH-Wert der anwendungsfertig verdünnten Reinigungslösung beträgt vorzugsweise wenigstens 11, weiter vorzugsweise wenigstens 11,5, weiter vorzugsweise wenigstens 12, weiter vorzugsweise wenigstens 12,5. Das Reinigungsmittel enthält bevorzugt Alkalihydroxide wie Natriumhydroxid oder bevorzugt Kaliumhydroxid. Die Verwendung von Kaliumhydroxid erleichtert das Bereitstellen eines Reinigungsmittels in Form eines Konzentrats, da Kaliumhydroxidlösungen bei niedrigen Temperaturen weniger zur Auskristallisation neigen als Natriumhydroxidlösungen.

Der bevorzugte Alkalihydroxidgehalt in der anwendungsfertig verdünnten Reinigungs-/Desinfektionslösung beträgt 200 bis 10.000 ppm, weiter vorzugsweise 200 bis 5.000 ppm, weiter vorzugsweise 200 bis 2.000 ppm. Die Angabe ppm bezieht sich auf Gewichtsteile.

Das Reinigungsmittel kann zusätzlich Alkanolamine enthalten.

Der Gehalt an kationischen Tensiden in der anwendungsfertig verdünnten Lösung beträgt vorzugsweise 15 bis 500 ppm, weiter vorzugsweise 15 bis 100 ppm, weiter vorzugsweise 15 bis 50 ppm. Nichtionische Tenside sind in der anwendungsfertig verdünnten Lösung vorzugsweise 15 bis 500 ppm, weiter vorzugsweise 15 bis 200 ppm, weiter vorzugsweise 25 bis 100 ppm enthalten. Der Gehalt an amphoteren Tensiden in der anwendungsfertig verdünnten Lösung beträgt vorzugsweise 50 bis 1.000 ppm, weiter vorzugsweise 100 bis 500 ppm, weiter vorzugsweise 150 bis 300 ppm.

Als kationische Tenside sind quarternäre Amoniumverbindungen besonders bevorzugt. Diese wirken im Rahmen der erfindungsgemäßen Kombination auch bei sehr niedrigen Anwendungskonzentrationen mikrobiozid.

Durch Zusatz von Tensiden zu der hochalkalischen Reinigerlösung kann die Oberflächen- und Grenzflächenspannung deutlich reduziert werden. Grundsätzlich sind nichtionische Tenside wie bspw. Fettalkohole am besten zur Verminderung der Oberflächenspannung einer wässrigen Lösung geeignet. Sie haben den zusätzlichen Vorteil, dass sie wenig schäumen und somit die unerwünschte Schaumbildung bei der Reinigung medizinischer Instrumente verhindern oder vermindern. Eine Schaumbildung kann insbesondere den Umwälzpumpendruck in der Spülmaschine reduzieren, die Reinigung bspw. englumiger Schläuche von Endoskopen oder dergleichen beeinträchtigen.

Die anwendungsfertig verdünnte Reinigerlösung weist vorzugsweise eine Oberflächenspannung von weniger als 50mN/m, vorzugsweise weniger als 40mN/m, weiter vorzugsweise weniger als 35 mN/m auf. Die Oberflächenspannung wird bestimmt nach der sogenannten Bügel-Ring-Methode nach DIN 53993. Ein weiterer Aspekt der Erfindung ist die Vermeidung oder Verminderung der sogenannten Redeposition von Verunreinigungen auf den Instrumenten. Der Begriff Redeposition bezeichnet die Wiederablagerung einer bereits von einer kontaminierten Oberfläche entfernten Verunreinigung auf einer anderen, möglicherweise vorher nicht kontaminierten Oberfläche des zu reinigenden Instruments.

Bereits die im Rahmen der Erfindung vorgesehene Verwendung von Tensiden verhindert die Redeposition, da die Tenside abgelöste Verunreinigungen emulgieren können und damit in der wässrigen Lösung in Schwebe halten. Bevorzugt ist im Rahmen der Erfindung zur Vermeidung oder Verringerung der Redeposition, dass das Reinigungsmittel zusätzlich Härtedispergatoren enthält. Als Härtedispergatoren können bspw. Phosphate und Polyphosphate, Komplex- oder Chelatbildner oder andere sogenannte Builder verwendet werden. Härtedispergatoren unterstützen die emulgierende Wirkung der Tenside und tragen somit zur Verhinderung der Redeposition bei.

Ein wichtiger Aspekt der Erfindung ist deren Eignung zur routinemäßigen, maschinellen Reinigung und Desinfektion. Für eine solche routinemäßige Reinigung werden im Stand der Technik üblicherweise schwachsaure oder schwachalkalische (bspw. enzymatische) Reiniger verwendet, da stark alkalische Lösungen zu einer erhöhten Beanspruchung oder Korrosion und damit Verschleiß von verschiedenen Materialien und Oberflächen führen können, die bei medizinischen Instrumenten und Apparaten verwendet werden. Problematisch in dieser Hinsicht sind bspw. Silikonelastomere, verchromte Instrumente, Lötverbindungen aus Silber und Zinn, Klebverbindungen und Dichtungsmaterialien, Kunststoffüberzüge wie bspw. Farbcodierungen, Glasfaserlichtleiter und optische Oberflächen mit Antireflexvergütung. Besonders problematisch sind Aluminiumoberflächen, insbesondere eloxierte Aluminiumoberflächen, da alkalische Lösungen diesen gegenüber eine besondere Aggressivität aufweisen. Die genannte Problematik tritt bspw. besonders auf bei der Reinigung von Endoskopen und deren Bestandteilen, da hier die zu reinigenden Oberflächen eine große Materialvielfalt aufweisen.

Bei einer bevorzugten Ausführungsform der Erfindung enthält daher das Reinigungsmittel zusätzlich Korrosionsinhibitoren. Darunter ist jeder Stoff zu verstehen, der in der alkalischen Lösung deren Angriff auf Oberflächen, insbesondere metallischen Oberflächen wie Aluminium oder eloxiertes Aluminium, hemmt. Geeignete Inhibitoren sind bspw. polymere Silicate wie bspw. Wasserglas, Phosphorsäureester oder dergleichen. Geeignete Phosphorsäureester sind Mono- und/oder Diester der Phosphorsäure mit aliphatischen Alkolholen der Kettenlänge C₁ bis C₂₂ und/oder aliphatischen Diolen und/oder aliphatischen Polyolen der Kettenlänge C₂ bis C₂₂. Erfindungsgemäß erhält man so trotz der Verwendung hochalkalischer Reinigerlösungen eine schonende Einwirkung auf bspw. eloxierte Aluminiumoberflächen.

Erfindungsgemäß wird aus den Bestandteilen des Reinigungsmittels vorzugsweise ein flüssiges Konzentrat formuliert, das mit Wasser zu der anwendungsfertigen Reinigungslösung verdünnt werden kann. In diesem Konzentrat liegt der Alkaligehalt (berechnet als KOH) vorzugsweise zwischen 2 und 30 Gew.-%, weiter vorzugsweise 15 und 26 Gew.-%. Der Tensidgehalt liegt bevorzugt zwischen 2 und 25 Gew.-%, weiter vorzugsweise 2 und 15 Gew.-%, weiter vorzugsweise 5 und 15 Gew.-%, weiter vorzugsweise 5 und 10 Gew.-%. Dieses Konzentrat wird vorzugsweise in Konzentrationen von 0,5 bis 5, vorzugsweise 0,5 bis 2, besonders bevorzugt 0,5 bis 1,5 Vol.-% mit Wasser zu einer gebrauchsfertigen Lösung angesetzt.

Wie bereits erwähnt, kann das Konzentrat wenigstens einen Komplexbildner, insbesondere Chelatbildner, enthalten. Die Komplexbildner dienen der Wasserenthärtung und können durch Komplexieren von Erdalkalionen die Reinigungswirkung gegenüber Kalkseifen verbessern. Bei den Komplexbildnern kann es sich um Homo-, Co- oder Terpolymere auf der Basis von Acrylsäure oder deren Alkalisalzen handeln, ferner um Phosphonsäuren bzw. deren Alkalisalze, wie bspw. 1-Hydroxyethan-1,1-diphosphonsäure, Aminotrismethylenphosphonsäure, Ethylendiaminotetrakismethylenphosphonsäure, Phosphonobutantricarbonsäure; Weinsäure, Citronensäure und Glukonsäure; ferner Nitrilotriessigsäure oder Ethylendiaminotetraessigsäure bzw. deren Salze.

Das Konzentrat kann Nitrilotriessigsäure und/oder ein Salz dieser Säure, besonders bevorzugt deren Trinatriumsalz, enthalten. Der NTA-Zusatz ist vorteilhaft, wenn das Konzentrat mit stark mineralstoffhaltigem (hartem) Wasser zu einer gebrauchsfertigen Lösung angesetzt werden soll.

Bei Bedarf können Konfektionierhilfsmittel (Lösungsvermittler) zugegeben werden wie bspw. Natriumcumolsulfonat, Natriumtoluolsulfonat, Natriumxylolosulfonat, Harnstoff, Glykole, insbesondere Polypropylenglykole und Polyethylenglykole, Methylacetamid und Fettalkohole, wie bspw. Cetylalkohol.

Die Aufzählung möglicher Inhaltsstoffe ist nicht abschlie-βend. Es können zusätzlich bspw. Netzmittel, Emulgatoren, schaumbremsende Mittel oder dergleichen zugesetzt werden. Vorteilhaft ist beispielsweise der Zusatz von N-Acylglutamat als Netzmittel.

Die Einwirkzeit des Reinigungsmittels beträgt erfindungsgemäß vorzugsweise 1 bis 60 min, weiter vorzugsweise 1 bis 30 min, weiter vorzugsweise 5 bis 30 min, weiter vorzugsweise 10 bis 20 min. Insbesondere beim Reinigen/Desinfizieren von Geschirr können auch kurze Einwirkzeiten von bspw. 30 s bis 5 min, insbesondere 60 s bis 3 min vorgesehen sein. Vor und/oder nach der Einwirkung des erfindungsgemäß verwendeten Reinigungsmittels können weitere Vorreinigungs-, Reinigungs-, Nachspül- oder Klarspül- oder Desinfektionsschritte vorgesehen werden. Bevorzugt ist es, zunächst ein Vorspülen zur Entfernung grober Verunreinigungen vorzunehmen, dann eine erfindungsgemäße Reinigung/Desinfektion, gefolgt von einem Nachspülen mit Wasser zur Entfernung von Reinigungsmittelresten.

Die Reinigung wird erfindungsgemäß bevorzugt bei einer Temperatur von Raumtemperatur bis 93°C, weiter vorzugsweise 40 bis 93°C, weiter vorzugsweise 50 bis 80°C, besonders bevorzugt 50 bis 60°C durchgeführt. Ebenfalls bevorzugt ist ein Temperaturbereich von Raumtemperatur (18°C) bis 50°C bzw. Raumtemperatur bis 40°C.

Bei der maschinellen Reinigung medizinischer Instrumente sind besonders bevorzugt Temperaturen von 50 bis 60°C, insbesondere etwa 55°C, und eine Einwirkzeit von 10 bis 20 min, vorzugsweise etwa 10 min. Das Reinigerkonzentrat gemäß dem nachfolgenden Beispiel 1 wird im Rahmen der maschinellen Reinigung bevorzugt in einer Anwendungskonzentration von etwa 1 bis 2 Vol.-% eingesetzt.

Gegenstand der Erfindung ist ferner ein Verfahren gemäβ Anspruch 22 zur maschinellen Reinigung und/oder Desinfektion von Gegenständen wie bspw. medizinischen und/oder chirurgischen Instrumenten und Apparaten oder Geschirr. Dieser Reinigungs- bzw. Desinfektionsschritt a) kann der erste Schritt des erfindungsgemäßen Verfahrens sein. Die verunreinigten Instrumente werden also ohne einen vorgeschalteten Reinigungsschritt unter sog. "dirty conditions" gereinigt und gleichzeitig desinfiziert. Diese Vorgehensweise hat den besonderen Vorteil, dass aus der Spülmaschine keinerlei kontaminiertes Abwasser abgelassen wird. Wenn dagegen in dem ersten oder den ersten Schritten des Verfahrens lediglich eine Reinigung ohne Inaktivierung der Mikroorganismen erfolgt, tritt kontaminiertes Abwasser aus der Spülmaschine aus und muss ggf. nachbehandelt werden, bevor es in die Kanalisation abgelassen werden kann. Bei der Inaktivierung der Mikroorganismen unter dirty conditions kann sich an diesen ersten Schritt a) beispielsweise ein Nachspülen und anschließendes Trocknen anschließen.

Erfindungsgemäß ist es ferner möglich, dass dem Reinigungs- bzw. Desinfektionsschritt a) ein oder mehrere weitere Vorspül- und/oder Reinigungsschritte vorgeschaltet sind. In diesem Fall wird in den ersten Schritten zunächst gereinigt, bevor dann unter sog. clean conditions die erfindungsgemäße Desinfektion erfolgt. Es ist besonders bevorzugt, wenn in dem vorgeschalteten Reinigungsschritt des gleiche Reinigungsmittel zur Herstellung der clean conditions verwendet wird wie in dem nachgelagerten Reinigungs- bzw. Desinfektionsschritt a). Es kann insbesondere in einer niedrigeren Konzentration (bevorzugt wenigstens um die Hälfte niedriger) verwendet werden, so dass in dem vorgelagerten Reinigungsschritt bei der niedrigeren Konzentration im wesentlichen gereinigt und erst in dem nachgelagerten Schritt erfindungsgemäß eine Abtötung/Inaktivierung der Mikroorganismen erfolgt. Besonders bevorzugte Parameter für den Reinigungs- bzw. Desinfektionsschritt a) sind Einwirktemperaturen von etwa 50 bis 60°C, insbesondere etwa 55°C, eine Einwirkzeit von 5 bis 15 Minuten, insbesondere etwa 10 Minuten, sowie eine Alkalikonzentration (berechnet als KOH) in der anwendungsfertig verdünnten Lösung von 0,05 bis etwa 0,2 Gew.-%, insbesondere etwa 0,1 Gew.-%. Die Gesamttensidkonzentration in der anwendungsfertig verdünnten Lösung kann gering sein und bspw. bei etwa 100 bis 200 ppm liegen. Die genannten Parameter stellen sich ein, wenn man ein Reinigungsmittelkonzentrat gemäß dem nachfolgenden Beispiel 1 im Verhältnis 1 zu 100 in Wasser löst.

Die erfindungsgemäße Verwendung bzw. das Verfahren ermöglichen eine wirksame Desinfizierung (Abtötung/Inaktivierung der genannten Mikroorganismen) von medizinischen und chirurgischen Instrumenten oder Geschirr ohne den Einsatz in Stand der Technik üblicher separater Desinfektionswirkstoffe wie beispielsweise Aktivchlor, Peroxiden, Aminwirkstoffen oder dergleichen. Es ist somit auch Gegenstand der Erfindung, dass das erfindungsgemäß eingesetzte Reinigungsmittel solche herkömmlichen Desinfektionswirkstoffe nicht enthält. Ferner ist es Gegenstand der Erfindung, dass auf eine im Stand der Technik übliche Thermodesinfektion (bspw. Spülen mit auf 93°C erhitztem Wasser) verzichtet werden kann.

### Beispiel 1

Ein Reinigungsmittelkonzentrat wird gemäß der nachfolgenden Tabelle 1 zubereitet. Die Mengen der einzusetzenden Ausgangsstoffe sind in Gewichtsteilen angegeben. (Rest auf 100 Gewichtsteile Wasser). Natriumalkylaminodipropionat ist ein amphoteres Tensid, Bardac LF eine quarternäre Ammoniumverbindung (QAV) und der Fettalkohol ein nichtionisches Tensid (n-Tensid).

| | |
|---|---|
| Kaliumtripolyphosphat | 21,39 |
| Kaliumhydroxid | 10,00 |
| Natriumalkylaminodipropionat | 2,40 |
| Fettalkohol, C10/12, 4EO, 4-5PO¹ | 0,50 |
| Bardac LF² | 0,25 |
| Natriumwasserglas | 27,90 |

| | |
|---|---|
| ¹Block-Copolymer aus C10/C12-Fettalkoholen mit 4 Ethylenoxid- und 4-5 Propylenoxideinheiten. ²Kationisches Tensid (Dioctyldimethylammoniumchlorid) | |

### Beispiele 2 bis 19

Die Zusammensetzung des Konzentrats des Beispiels 1 wurde gemäß den Angaben in der nachfolgenden Tabelle 2 variiert. Die Tabelle 2 nennt nur die Änderungen gegenüber der Rezeptur des Beispiels 1, alle nicht genannten Bestandteile sind in den Beispielen 2 bis 19 in dem gleichen Mengenanteil enthalten wie in Beispiel 1. In jedem Fall wird mit Wasser auf 100 Gewichtsteile ergänzt. Bei den Beispielen 14 bis 16 enthält das Konzentrat zusätzlich 2 Gew.-% Isopropanol als Lösevermittler für die abgewandelten und schwerer löslichen amphoteren Tenside.

**Tabelle 2**

| | |
|---|---|
| Beispiel 2 | ohne Bardac LF (QAV) |
| Beispiel 3 | ohne Fettalkohol (n-Tensid) |
| Beispiel 4 | ohne Natriumalkylaminodipropionat (ampho-Tensid) |
| Beispiel 5 | Kaliumhydroxid-Gehalt auf 5 Gew.-% reduziert |
| Beispiel 6 | Bardac LF ersetzt durch Bardac 2270E(Didecyldimethylammoniumchlorid) |
| Beispiel 7 | Bardac LF-Gehalt reduziert auf 0,25 Gew.-% |
| Beispiel 8 | Bardac LF-Gehalt erhöht auf 0,375 Gew.-% |
| Beispiel 9 | Fettalkohol-Gehalt reduziert auf 0,25 Gew.-% |
| Beispiel 10 | Fettalkohol-Gehalt erhöht auf 0,75 Gew.-% |
| Beispiel 11 | Fettalkohol, C10/12, 4EO, 4-5PO ersetzt durch Oleyl-Cetylalkohol 5 EO (Eumulgin EP 5 L-V) |
| Beispiel 12 | Fettalkohol, C10/12, 4EO, 4-5PO ersetzt durch endgruppenverschlossenes C12/18 5EO (Dehypon LT 054) |
| Beispiel 13 | Fettalkohol, C10/12, 4EO, 4-5PO ersetzt durch N-Octylpyrrolidon |
| Beispiel 14 | Bardac LF ersetzt durch N,N-Didecyl-N-methyl-poly-(oxyethyl)-ammoniumpropionat (Bardap 26) |
| Beispiel 15 | Bardac LF ersetzt durch Lauryldimethylbenzy-lammoniumchlorid / Lauryldimethylethylbenzy-lammoniumchlorid (BTC 2125 M) |
| Beispiel 16 | Bardac LF ersetzt durch N-Alkyl(C12/14)-N-benzyl-N,N-dimethylammoniumchlorid (Barquat LB 50) |
| Beispiel 17 | Natriumalkylaminodipropionat ersetzt durch Alkylaminoessigsäuregemisch (Ampholyt 51/27) |
| Beispiel 18 | Natriumalkylaminodipropionat ersetzt durch Na-Capr.amphppropionat (Rewoteric AM-VSF) |
| Beispiel 19 | Natriumalkylaminodipropionat ersetzt durch Fettsäureamidopropylbetain (Tegotens B 810) |

### Vergleichsbeispiele 1 bis 6

Nicht erfindungsgemäße Reinigungsmittelkonzentrate werden gemäß der nachfolgenden Tabelle 3 zubereitet. Die Tabelle 3 nennt nur die Änderungen gegenüber der Rezeptur des Beispiels 1, alle nicht genannten Bestandteile sind in den Vergleichsbeispielen 1 bis 6 in dem gleichen Mengenanteil enthalten wie in Beispiel 1. In jedem Fall wird mit Wasser auf 100 Gewichtsteile ergänzt.

**Tabelle 3**

| | |
|---|---|
| Vergleichsbeispiel 1 | ohne Tenside (alle 3 Tenside weggelassen) |
| Vergleichsbeispiel 2 | ohne Bardac LF und Fettalkohol |
| Vergleichsbeispiel 3 | ohne Fettalkohol und Natriumalkylaminodipropionat |
| Vergleichsbeispiel 4 | ohne Bardac LF und Natriumalkylaminodipropionat |
| Vergleichsbeispiel 5 | Kaliumhydroxid-Gehalt auf 1 Gew.-% reduziert |
| Vergleichsbeispiel 6 | ohne Kaliumhydroxid |

### Beispiel 20

Die Reinigungsmittelkonzentrate gemäß Beispielen 1 bis 19 und Vergleichsbeispielen 1 bis 6 werden auf bakterizide Wirksamkeit geprüft. Die nachstehend genannten Normen und insbesondere die dort beschriebenen Bedingungen und Verfahren zur Prüfung der Wirksamkeit werden durch Bezugnahme darauf ausdrücklich zum Gegenstand auch der vorliegenden Anmeldung gemacht. Bei der Prüfung der Abtötung/Inaktivierung von Bakterien wurde der Reduktionsfaktor der in Tabelle 4 genannten Mikroorganismen gemessen einer wässrigen Lösung der Konzentrate bei der angegebenen Einwirkdauer und Temperatur. Die eingesetzte Konzentration des Reinigungsmittels ist in Vol.-% angegeben. Diese Konzentrationen, Einwirkdauer und Temperaturen entsprechen in etwa den typischen Bedingungen bei einer maschinellen Reinigung. Die erzielten Reduktionsfaktoren sind in dekadischen Logarithmenstufen (Log-Stufen) angegeben.

**Tabelle 4**

| | **Wirkstoffkonzentration in 1 %iger Lösung [ppm]** | | | | **Mykobakterium terrae Prüfung nach Standardmethoden DGHM, Prüftemperatur 55°C, Einwirkzeit 10 min geringe Belastung** | | | | **Mykobakterium terrae Prüfung nach Standardmethoden DGHM, Prüftemperatur 55°C, Einwirkzeit 10 min hohe Belastung** | | | | **Enterococcus hirae Prüfung nach DIN EN 13727, Prüftemperatur 20°C, Einwirkzeit 5 min geringe Belastung** | |
|---|---|---|---|---|---|---|---|---|---|---|---|---|---|---|
| | **QAV** | **n-Tensid** | **ampho-T.** | **KOH** | **1,0 Vol.-%** | **1,5 Vol.-of** | **2,0 Vol.-%** | **2,5 Vol: %** | **1,0 Vol.-%** | **1,5 Vol.-%** | **2,0 Vol: %** | **2,5 Vol.-%** | **1,0 Vol.-%** | **2.0 Vol.-%** |
| Beispiel 1 | 25 | 50 | 240 | 1000 | 3,22 | 4,10 | 6,10 | | 3,41 | 4,66 | 4,54 | 5,14 | - | - |
| Beispiel 2 | - | 50 | 240 | 1000 | - | 3,38 | 3,40 | - | - | 3,42 | 3,78 | 3,79 | - | - |
| Beispiel 3 | 25 | - | 240 | 1000 | - | 4,15 | 3,80 | - | - | 3,19 | 3,36 | 3,84 | - | - |
| Beispiel 4 | 25 | 50 | - | 1000 | - | 3,61 | 3,92 | - | - | 3,38 | 3,54 | 3,66 | - | - |
| Beispiel 5 | 25 | 50 | 240 | 500 | - | 3,80 | 4,32 | - | - | 3,80 | 4,19 | 4,36 | - | - |
| Beispiel 6 | 25 | 50 | 240 | 1000 | - | 4,80 | 4,80 | - | - | 4,36 | 4,54 | 4,66 | - | - |
| Beispiel 7 | 12,5 | 50 | 240 | 1000 | 1,52 | 3,58 | 4,06 | - | 2,33 | 3,28 | 3,38 | - | - | - |
| Beispiel 8 | 37,5 | 50 | 240 | 1000 | 3,30 | 3,76 | 3,95 | - | 3,55 | 3,71 | 4,03 | - | - | - |
| Beispiel 9 | 25 | 25 | 240 | 1000 | 3,07 | 3,80 | 4,06 | - | 3,27 | 3,57 | 3,88 | - | - | - |
| Beispiel 10 | 25 | 75 | 240 | 1000 | 3,47 | 3,70 | 3,99 | - | 3,43 | 3,65 | 4,14 | - | - | - |
| Vergleichsbeispieil 1 | - | - | - | 1000 | - | 1,67 | 1,87 | - | - | 1,94 | 2,04 | 2,10 | - | - |
| Vergleichsbeispiel 2 | - | - | 240 | 1000 | < 1,22 | < 1,22 | < 1,22 | - | < 1,05 | < 1,05 | < 1,05 | - | - | - |
| Vergleichsbeispiel 3 | 25 | - | - | 1000 | < 1,22 | 1,77 | 1,93 | - | < 1,05 | 1,59 | 1,60 | - | - | - |
| Vergleichsbeispiel 4 | - | 50 | - | 1000 | 1,74 | 1,86 | 1,96 | - | < 1,05 | 1,61 | 1,80 | - | - | - |
| Vergleichsbeispiel 5 | 25 | 50 | 240 | 100 | - | - | - | - | - | - | - | - | 1,17 | 4,74 |
| Vergleichsbeispiel 6 | 25 | 50 | 240 | - | - | - | - | - | - | - | - | - | 0,55 | 2,76 |
| Beispiel 11 | 25 | 50 | 240 | 1000 | - | - | - | - | - | - | - | - | 2,62 | > 5,23 |
| Beispiel 12 | 25 | 50 | 240 | 1000 | - | - | - | - | - | - | - | - | 3,23 | > 5,23 |
| Beispiel 13 | 25 | 50 | 240 | 1000 | - | - | - | - | - | - | - | - | 3,83 | > 5,23 |
| Beispiel 14 | 25 | 50 | 240 | 1000 | - | - | - | - | - | - | - | - | 4,39 | > 5,23 |
| Beispiel 15 | 25 | 50 | 240 | 1000 | - | - | - | - | - | - | - | - | > 5,23 | > 5,23 |
| Beispiel 16 | 25 | 50 | 240 | 1000 | - | - | - | - | - | - | - | - | > 5,23 | > 5,23 |
| Beispiel 17 | 25 | 50 | 240 | 1000 | - | - | - | - | - | - | - | - | > 5,23 | > 5,23 |
| Beispiel 18 | 25 | 50 | 240 | 1000 | - | - | - | - | - | - | - | - | 4,99 | > 5,23 |
| Beispiel 19 | 25 | 50 | 240 | 1000 | - | - | - | - | - | - | - | - | 4,19 | 4,51 |

In der Tabelle 4 bedeutet "geringe Belastung", dass die Eiweißbelastung der verwendeten Prüfsuspensionen gering ist (0,03% Rinderserumalbumin). Dies entspricht der Inaktivierung der Mikroorganismen unter sog. clean conditions, bei denen vor der Desinfektion ein separater Reinigungsschritt vorgenommen worden ist. Entsprechend bedeutet "hohe Belastungen" eine hohe Belastung mit Eiweißrückständen (0,3% Rinderserumalbumin und 0,3% Schaf-Erythrozyten), dies entspricht der Desinfektion (und gleichzeitigen Reinigung) unter sog. dirty conditions, bei denen keine vorgeschaltete separate Reinigung oder Vorspülung erfolgt.

Die Versuche der Tabelle 4 zeigen, dass sich eine Reduktion der Mikroorganismen um wenigstens den Faktor 10³ (3 Log-Stufen) über eine breite Variation von Menge und Art der Inhaltsstoffe erreichen lässt. Die Vergleichsbeispiele wiederum belegen, dass eine solche Inaktivierung dann nicht eintritt, wenn weniger als zwei Tenside aus den genannten drei Tensidgruppen vorhanden sind oder die Alkalität unzureichend ist. Eine noch bessere Inaktivierung der Mikroorganismen ergibt sich bei Kombination aller drei Tensidgruppen.

Im Rahmen der Erfindung ist eine Reduktion der Mikroorganismen um wenigstens 3 Log-Stufen bevorzugt. Weiter bevorzugt ist eine Reduktion um wenigstens 4 Log-Stufen. Diese Reduktion um 4 Log-Stufen kann insbesondere bei Funghi bevorzugt sein. Weiter bevorzugt ist eine Reduktion um 5 Log-Stufen, insbesondere bei Bakterien.

### Beispiel 21

In diesem Beispiel wird die Wirksamkeit der Rezeptur des Beispiels 1 gegenüber verschiedenen Mikroorganismen und bei verschiedenen Einwirkzeiten und -konzentrationen gezeigt. In der nachfolgenden Tabelle 5 entsprechen die Angaben zur Schmutzbelastung der Prüfkörper und der eingesetzten Reinigerkonzentrationen den in Tabelle 4 verwendeten Abkürzungen. Aus der Tabelle ist ersichtlich, dass Beispiel 1 eine Inaktivierung um wenigstens den Faktor 10⁴ der Testfungi candida albicans und Aspergillus niger bewirkt.

**Tabelle 5**

| **Mykobakterium terrae** | | |
|---|---|---|
| Prüfung nach DIN EN 14348, Prüftemperatur 55°C, Einwirkzeit 10 min | | |
| **geringe Bel.** | **1,0 Vol.-%** | 5,60 |
| | **1,5 Vol.-%** | 6,18 |
| | **2,0 Vol.-%** | 6,78 |
| **hohe Bel.** | **1,5 Vol.-%** | 5,32 |
| | **2,0 Vol.-%** | 5,08 |

| **E. hirae** | | |
|---|---|---|
| Prüfung nach DIN EN 13727, Prüftemperatur 55°C, Einwirkzeit 5 min | | |
| **geringe Bel.** | **1,0 Vol.-%** | >5,23 |
| **hohe Bel.** | **1,0 Vol.-%** | > 5,23 |

| **Ps. aerug.** | | |
|---|---|---|
| Prüfung nach DIN EN 13727, Prüftemperatur 55°C, Einwirkzeit 5 min | | |
| **geringe Bel.** | **1,0 Vol.-%** | > 5, 04 |
| **hohe Bel.** | **1,0 Vol.-%** | > 5,04 |

| **Staph. aur.** | | |
|---|---|---|
| Prüfung nach DIN EN 13727, Prüftemperatur 55°C, Einwirkzeit 5 min | | |
| **geringe Bel.** | **1,0 Vol.-%** | > 5,28 |
| **hohe Bel.** | **1,0 Vol.-%** | > 5,28 |

| **C. albic.** | | |
|---|---|---|
| Prüfung nach DIN EN 13624, Prüftemperatur 55°C, Einwirkzeit 5 min bei Prüfkonzentration 1,0 Vol.-%; 10 min bei Prüfkonzentration 2,0 Vol.-% | | |
| **ger. Bel.** | **1,0 Vol.-%** | > 4,15 |
| | **2,0 Vol.-%** | > 4,26 |
| **hohe Bel.** | **2,0 Vol.-%** | > 4,15 |

| **Asp. niger** | | |
|---|---|---|
| Prüfung nach DIN EN 13624, Prüftemperatur 55°C, Einwirkzeit 5 min bei Prüfkonzentration 1,0 Vol.-%; 10 min bei Prüfkonzentration 2,0 Vol.-% | | |
| **ger. Bel.** | **1,0 Vol.-%** | 3,83 |
| | **2,0 Vol.-%** | > 4,20 |
| **hohe Bel.** | **2,0 Vol.-%** | > 4,28 |

### Beispiel 22

In diesem Beispiel werden zwei Programmabläufe zur Reinigung und Desinfektion von medizinischen und chirurgischen Instrumenten in einer üblichen Eintankspülmaschine angegeben.

Programmablauf 1:
- Vorreinigung mit Kaltwasser, 3 min,
- Reinigung mit einer 0,3 Vol.-%igen wässrigen Lösung der Rezeptur gemäß Beispiel 1 bei 55°C, Haltezeit 3 min,
- Reinigung/Desinfektion mit einer 1 Vol.-%igen wässrigen Lösung der Rezeptur gemäß Beispiel 1 bei 55°C, Haltezeit 10 min,
- Nachspülung mit Wasser unter Zudosierung eines sauren Neutralisationsmittels auf Basis Zitronensäure,
- Zwischenspülung mit Kaltwasser,
- Schlussspülung mit Wasser, Aufheizen auf 55°C mit Einwirkzeit von 1 min,
- Trocknung mit Heißluft.

Bei diesem Verfahrensablauf wird die Desinfektion unter sog. clean conditions durchgeführt, d.h. vor der eigentlichen Desinfektion wird zunächst eine Reinigung durchgeführt mit einer geringeren Konzentration des Reinigungsmittels gemäß Beispiel 1. Die im Programmablauf vorgesehene Kaltwasservorspülung kann optional ganz weggelassen werden. Die Nachspülung nach dem Desinfektionsschritt kann wahlweise auch ohne Neutralisationsmittel erfolgen, wenn hinreichend Wasser zum Abspülen der alkalischen Rezeptur gemäß Beispiel 1 eingesetzt wird.

Programmablauf 2:
- Vorreinigung mit Kaltwasser, 3 min,
- Reinigung/Desinfektion mit einer 1 Vol.-%igen wässrigen Lösung der Rezeptur gemäß Beispiel 1 bei 55°C, Haltezeit 10 min,
- Nachspülung mit Wasser unter Zudosierung eines sauren Neutralisationsmittels auf Basis Zitronensäure,
- Zwischenspülung mit Kaltwasser,
- Schlussspülung mit Wasser, Aufheizen auf 55°C mit Einwirkzeit von 1 min,
- Trocknung mit Heißluft.

Bei diesem Programmablauf erfolgt die Desinfektion unter dirty conditions, d.h. Reinigung und Desinfektion erfolgen in einem einzigen Schritt gleichzeitig. Optional kann die Kaltwasservorreinigung wegfallen. Dies hat bei der Reinigung unter dirty conditions den besonderen Vorteil, dass dann sämtliches aus der Spülmaschine austretendes Spülwasser nicht kontaminiert ist, da bereits die erste mit den verunreinigten Instrumenten in Berührung tretende Reinigerlösung desinfizierend wirkt und so Kontaminationen durch Mikroorganismen beseitigt.

Erfindungsgemäß ist eine abschließende Thermodesinfektion in keinem der Programmabläufe erforderlich. Dies verkürzt die Programmabläufe, da ein Aufheizen auf die Thermodisinfektionstemperatur von bspw. 93°C zeit kostet, ferner wird Energie eingespart sowie der Verschleiß empfindlicher Instrumente (insbesondere Kunststoff- und Gummiteile) durch eine thermische Belastung vermindert.

### Beispiel 23

Zur Prüfung der Materialschonung eloxierter Aluminiumoberflächen werden eloxierte Aluminiumplatten in der Spülmaschine G7736 der Firma Miele für 10 min bei 55°C einem Reinigungsmedium ausgesetzt. Es werden sowohl neue farblos als auch blau eloxierte Aluminiumplatten verwendet. Das Reinigungsmedium ist 0,1 M NaOH mit einem pH-Wert von 12,7 bzw. eine 1 Vol.-%ige Reinigerlösung des Reinigungsmittelkonzentrats gemäß Beispiel 1. Anschließend werden die Platten visuell untersucht. Bei den mit NaOH behandelten Platten ist die Eloxalschicht deutlich abgetragen. Im Unterschied dazu weisen die mit dem Reiniger behandelten Platten keine sichtbaren Schädigungen der Eloxalschicht auf.

## Patentansprüche

1. Verwendung eines Reinigungsmittels, das wenigstens zwei verschiedene Tenside enthält, ausgewählt aus wenigstens zwei der drei Gruppen kationische, nichtionische und amphotere Tenside, und anwendungsfertig verdünnt in wässriger Lösung einen pH-Wert von wenigstens 10,5 aufweist, zur Abtötung/Inaktivierung von Mikroorganismen ausgewählt aus der Gruppe bestehend aus Bakterien, Viren und Pilzen bei der maschinellen Desinfektion von Gegenständen.

2. Verwendung nach Anspruch 1, **dadurch gekennzeichnet, dass** die Gegenstände medizinische und/oder chirurgische Instrumente oder Apparate sind.

3. Verwendung nach Anspruch 1, **dadurch gekennzeichnet, dass** die Gegenstände Geschirr sind.

4. Verwendung nach Anspruch 3, **dadurch gekennzeichnet, dass** das Geschirr Glasgeschirr ist.

5. Verwendung nach einem der Ansprüche 1 bis 4, **dadurch gekennzeichnet, dass** der pH-Wert wenigstens 11, vorzugsweise wenigstens 11,5, vorzugsweise wenigstens 12, weiter vorzugsweise wenigstens 12,5 beträgt.

6. Verwendung nach einem der Ansprüche 1 bis 5, **dadurch gekennzeichnet, dass** das Reinigungsmittel Alkalihydroxide enthält.

7. Verwendung nach Anspruch 6, **dadurch gekennzeichnet, dass** als Alkalihydroxid KOH verwendet wird.

8. Verwendung nach Anspruch 6 oder 7, **dadurch gekennzeichnet, dass** in der anwendungsfertig verdünnten Lösung der Alkalihydroxidgehalt 200 - 10.000 ppm, vorzugsweise 200 bis 5.000 ppm, weiter vorzugsweise 200 bis 2.000 ppm beträgt.

9. Verwendung nach einem der Ansprüche 1 bis 8, **dadurch gekennzeichnet, dass** das Reinigungsmittel Alkanolamine enthält.

10. Verwendung nach einem der Ansprüche 1 bis 9, **dadurch gekennzeichnet, dass** das Reinigungsmittel kationische, nichtionische und amphotere Tenside enthält.

11. Verwendung nach einem der Ansprüche 1 bis 10, **dadurch gekennzeichnet, dass** in der anwendungsfertig verdünnten Lösung der Gehalt an kationischen Tensiden 15 - 500 ppm, vorzugsweise 15 - 100 ppm, weiter vorzugsweise 15 - 50 ppm beträgt.

12. Verwendung nach einem der Ansprüche 1 bis 11, **dadurch gekennzeichnet, dass** in der anwendungsfertig verdünnten Lösung der Gehalt an nichtionischen Tensiden 15 - 500 ppm, vorzugsweise 15 - 200 ppm, weiter vorzugsweise 25 - 100 ppm beträgt.

13. Verwendung nach einem der Ansprüche 1 bis 12, **dadurch gekennzeichnet, dass** in der anwendungsfertig verdünnten Lösung der Gehalt an amphoteren Tensiden 50 - 1.000 ppm, vorzugsweise 100 - 500 ppm, weiter vorzugsweise 150 - 300 ppm beträgt.

14. Verwendung nach einem der Ansprüche 1 bis 12, **dadurch gekennzeichnet, dass** die kationischen Tenside quartenäre Ammoniumverbindungen sind.

15. Verwendung nach einem der Ansprüche 1 bis 14, **dadurch gekennzeichnet, dass** das Reinigungsmittel anwendungsfertig verdünnt eine Oberflächenspannung von weniger als 50 mN/m, vorzugsweise weniger als 40 mN/m, weiter vorzugsweise weniger als 35 mN/m aufweist.

16. Verwendung nach einem der Ansprüche 1 bis 15, **dadurch gekennzeichnet, dass** das Reinigungsmittel Härtedispergiermittel enthält.

17. Verwendung nach Anspruch 16, **dadurch gekennzeichnet, dass** das Reinigungsmittel Phosphate und/oder Polyphosphate enthält.

18. Verwendung nach einem der Ansprüche 1 bis 17, **dadurch gekennzeichnet, dass** das Reinigungsmittel Korrosionsinhibitoren enthält.

19. Verwendung nach Anspruch 18, **dadurch gekennzeichnet, dass** die Korrosionsinhibitoren ausgewählt sind aus der Gruppe besteht aus polymeren Silicaten und Phosphorsäureestern.

20. Verwendung nach einem der Ansprüche 1 bis 19, **dadurch gekennzeichnet, dass** die Einwirkzeit des Reinigungsmittels 1 bis 60 min, vorzugsweise 1 bis 30 min, weiter vorzugsweise 5 bis 30 min, weiter vorzugsweise 10 bis 20 min beträgt.

21. Verwendung nach einem der Ansprüche 1 bis 20, **dadurch gekennzeichnet, dass** die Reinigung bei einer Temperatur von Raumtemperatur bis 93°C, vorzugsweise 40 bis 93°C, weiter vorzugsweise 50 bis 80°C, weiter vorzugsweise 50 bis 60°C stattfindet.

22. Verfahren zur maschinellen Reinigung und/oder DesInfektion von Gegenständen, wobei in wenigstens einem Reinigungs- und/oder Desinfektionsschritt a) durch ein Reinigungsmittel, das wenigstens zwei verschiedene Tenside enthält ausgewählt aus wenigstens zwei der drei Gruppen kationische, nichtionische und amphotere Tenside und des anwendungsfertig verdünnt in wässriger Lösung einen pH-Wert von wenigstens 10,5 aufweist, eine Abtötung/Inaktivierung von Mikroorganismen ausgewählt aus der Gruppe bestehend aus Bakterien, Viren und Pilzen erfolgt.

23. Verfahren nach Anspruch 22, **dadurch gekennzeichnet, dass** der Reinigungs- und/oder Desinfektionsschritt a) der erste Schritt des Verfahrens ist.

24. Verfahren nach Anspruch 22, **dadurch gekennzeichnet, dass** dem Reinigungs- und/oder Desinfektionsschritt a) ein Reinigungsschritt vorgeschaltet ist.

25. Verfahren nach Anspruch 24, **dadurch gekennzeichnet, dass** in dem vorgeschalteten Reinigungsschritt das gleiche Reinigungsmittel verwendet wird wie in dem Reinigungs- und/oder Desinfektionsschritt a).

26. Verfahren nach Anspruch 25, **dadurch gekennzeichnet, dass** in dem vorgeschalteten Reinigungsschritt die Konzentration des Reinigungsmittels niedriger ist als in dem Reinigungs- und/oder Desinfektionsschritt a).

27. Verfahren nach Anspruch 26, **dadurch gekennzeichnet, dass** in dem vorgeschalteten Reinigungsschritt die Konzentration des Reinigungsmittels um wenigstens die Hälfte niedriger ist als in dem Reinigungs- und/oder Desinfektionsschritt a).

28. Verfahren nach einem der Ansprüche 22 bis 27, **dadurch gekennzeichnet, dass** es keinen Thermodesinfektionsschritt umfasst.

## Claims

1. Use of a cleaning agent which contains at least two different surfactants selected from at least two of the three groups of cationic, non-ionic and amphoteric surfactants, and, diluted ready-to-use in aqueous solution, has a pH value of at least 10.5, for destroying/inactivating microorganisms selected from the group comprising bacteria, viruses and fungi in the mechanical disinfection of articles.

2. Use according to Claim 1, **characterised in that** the articles are medical and/or surgical instruments or apparatus.

3. Use according to Claim 1, **characterised in that** the articles are tableware.

4. Use according to Claim 3, **characterised in that** the tableware is glass tableware.

5. Use according to any one of Claims 1 to 4, **characterised in that** the pH value is at least 11, preferably at least 11.5, preferably at least 12, further preferably at least 12.5.

6. Use according to any one of Claims 1 to 5, **characterised in that** the cleaning agent contains alkali hydroxides.

7. Use according to Claim 6, **characterised in that** KOH is used as alkali hydroxide.

8. Use according to Claim 6 or 7, **characterised in that**, in the diluted ready-to-use solution, the alkali hydroxide content is 200-10,000 ppm, preferably 200 to 5,000 ppm, further preferably 200 to 2,000 ppm.

9. Use according to any one of Claims 1 to 8, **characterised in that** the cleaning agent contains alkanolamines.

10. Use according to any one of Claims 1 to 9, **characterised in that** the cleaning agent contains cationic, non-ionic and amphoteric surfactants.

11. Use according to any one of Claims 1 to 10, **characterised in that**, in the diluted ready-to-use solution, the content of cationic surfactants is 15-500 ppm, preferably 15-100 ppm, further preferably 15-50 ppm.

12. Use according to any one of Claims 1 to 11, **characterised in that**, in the diluted ready-to-use solution, the content of non-ionic surfactants is 15-500 ppm, preferably 15-200 ppm, further preferably 25-100 ppm.

13. Use according to any one of Claims 1 to 12, **characterised in that**, in the diluted ready-to-use solution, the content of amphoteric surfactants is 50-1,000 ppm, preferably 100-500 ppm, further preferably 150-300 ppm.

14. Use according to any one of Claims 1 to 12, **characterised in that** the cationic surfactants are quaternary ammonium compounds.

15. Use according to any one of Claims 1 to 14, **characterised in that** the cleaning agent, diluted ready-to-use, has a surface tension of less than 50 mN/m, preferably less than 40 mN/m, further preferably less than 35 mN/m.

16. Use according to any one of Claims 1 to 15, **characterised in that** the cleaning agent contains hardness dispersants.

17. Use according to Claim 16, **characterised in that** the cleaning agent contains phosphates and/or polyphosphates.

18. Use according to any one of Claims 1 to 17, **characterised in that** the cleaning agent contains corrosion inhibitors.

19. Use according to Claim 18, **characterised in that** the corrosion inhibitors are selected from the group comprising polymeric silicates and phosphoric acid esters.

20. Use according to any one of Claims 1 to 19, **characterised in that** the action time of the cleaning agent is 1 to 60 min, preferably 1 to 30 min, further preferably 5 to 30 min, further preferably 10 to 20 min.

21. Use according to any one of Claims 1 to 20, **characterised in that** the cleaning takes place at a temperature from room temperature to 93°C, preferably 40 to 93°C, further preferably 50 to 80°C, further preferably 50 to 60°C.

22. A method for the mechanical cleaning and/or disinfection of articles, wherein, in at least one cleaning and/or disinfection step a), destruction/inactivation of microorganisms selected from the group comprising bacteria, viruses and fungi is carried out by a cleaning agent which contains at least two different surfactants selected from at least two of the three groups of cationic, non-ionic and amphoteric surfactants and which, diluted ready-to-use in aqueous dilution, has a pH value of at least 10.5.

23. A method according to Claim 22, **characterised in that** the cleaning and/or disinfection step a) is the first step of the method.

24. A method according to Claim 22, **characterised in that** a cleaning step is provided upstream of the cleaning and/or disinfection step a).

25. A method according to Claim 24, **characterised in that**, in the upstream cleaning step, the same cleaning agent is used as in the cleaning and/or disinfection step a).

26. A method according to Claim 25, **characterised in that**, in the upstream cleaning step, the concentration of the cleaning agent is lower than in the cleaning and/or disinfection step a).

27. A method according to Claim 26, **characterised in that**, in the upstream cleaning step, the concentration of the cleaning agent is lower by at least half than in the cleaning and/or disinfection step a).

28. A method according to any one of Claims 22 to 27, **characterised in that** it comprises no thermal disinfection step.

## Revendications

1. Utilisation d'un détergent, qui contient au moins deux agents tensioactifs différents, choisis parmi au moins deux des trois groupes suivants :
- les agents tensioactifs cationiques,
- les agents non ioniques , et
- les agents amphotères,
et qui présente, à l'état dilué dans une solution aqueuse, prêt à l'emploi, une valeur pH d'au moins 10,5, afin d'éliminer/d'inactiver des micro-organismes choisis parmi le groupe constitué de bactéries, de virus et de champingnons dans le cas de la désinfection en machine d'objets.

2. Utilisation selon la revendication 1, **caractérisée en ce que** les objets sont des instruments ou des appareils médicaux et/ou chirurgicaux.

3. Utilisation selon la revendication 1, **caractérisée en ce que** les objets sont de la vaisselle.

4. Utilisation selon la revendication 3, **caractérisée en ce que** la vaisselle est de la vaisselle en verre.

5. Utilisation selon l'une quelconque des revendications 1 à 4, **caractérisée en ce que** la valeur pH est d'au moins 11, préférentiellement d'au moins 11,5, de manière plus préférentielle d'au moins 12, manière encore plus préférentiellement d'au moins 12,5.

6. Utilisation selon l'une quelconque des revendications 1 à 5, **caractérisée en ce que** le détergent contient des hydroxydes alcalins.

7. Utilisation selon la revendication 6, **caractérisée en ce que** l'hydroxyde alcalin est de l'hydroxyde de potassium (KOH).

8. Utilisation selon la revendication 6 ou 7, **caractérisée en ce que** la teneur en hydroxyde alcalin dans la solution diluée prête à l'emploi est comprise entre 200 et 10.000 ppm, de préférence entre 200 et 5.000 ppm, et de manière plus préférentielle entre 200 et 2.000 ppm.

9. Utilisation selon l'une quelconque des revendications 1 à 8, **caractérisée en ce que** le détergent contient des alcanolamines.

10. Utilisation selon l'une quelconque des revendications 1 à 9, **caractérisée en ce que** le détergent contient des agents tensioactifs cationiques, non ioniques et amphotères.

11. Utilisation selon l'une quelconque des revendications 1 à 10, **caractérisée en ce que** la teneur en agents tensioactifs cationiques dans la solution diluée prête à l'emploi est comprise entre 15 et 500 ppm, de préférence entre 15 et 100 ppm, et de manière plus préférentielle entre 15 et 50 ppm.

12. Utilisation selon l'une quelconque des revendications 1 à 11, **caractérisée en ce que** la teneur en agents tensioactifs non ioniques dans la solution diluée prête à l'emploi est comprise entre 15 et 500 ppm, de préférence entre 15 et 200 ppm, et de manière plus préférentielle entre 25 et 100 ppm.

13. Utilisation selon l'une quelconque des revendications 1 à 12, **caractérisée en ce que** la teneur en agents tensioactifs amphotères dans la solution diluée prête à l'emploi est comprise entre 50 et 1.000 ppm, de préférence entre 100 et 500 ppm, et de manière plus préférentielle entre 150 et 300 ppm.

14. Utilisation selon l'une quelconque des revendications 1 à 12, **caractérisée en ce que** les agents tensioactifs cationiques sont des composés d'ammonium quaternaires.

15. Utilisation selon l'une quelconque des revendications 1 à 14, **caractérisée en ce que** le détergent présente, à l'état dilué prêt à l'emploi une tension superficielle inférieure à 50 mN/m, de préférence inférieure à 40 mN/m, et de manière plus préférentielle inférieure à 35 mN/m.

16. Utilisation selon l'une quelconque des revendications 1 à 15, **caractérisée en ce que** le détergent contient des agents de dispersion de dureté.

17. Utilisation selon la revendication 16, **caractérisée en ce que** le détergent contient des phosphates et/ou des polyphosphates.

18. Utilisation selon l'une quelconque des revendications 1 à 17, **caractérisée en ce que** le détergent contient des inhibiteurs de corrosion.

19. Utilisation selon la revendication 18, **caractérisée en ce que** les inhibiteurs de corrosion sont choisis parmi le groupe constitué de silicates polymères et d'esters d'acide phosphorique.

20. Utilisation selon l'une quelconque des revendications 1 à 19, **caractérisée en ce que** la durée d'action du détergent est comprise entre 1 et 60 min, de préférence entre 1 et 30 min, de manière plus préférentielle entre 5 et 30 min, et de manière encore plus préférentielle entre 10 et 20 min.

21. Utilisation selon l'une quelconque des revendications 1 à 20, **caractérisée en ce que** le nettoyage a lieu à une température comprise entre la température ambiante et 93°C, de préférence à une température comprise entre 40 et 93 °C, de manière plus préférentielle à une température comprise entre 50 et 80 °C, et de manière encore plus préférentielle à une température comprise entre 50 et 60 °C.

22. Procédé de nettoyage et/ou désinfection en machine d'objets, sachant, lors d'au moins une étape de nettoyage et/ou de désinfection a), qu'un détergent, qui contient au moins deux agents tensioactifs différents, choisis parmi au moins deux des trois groupes suivants :
- les agents tensioactifs cationiques,
- les agents non-ioniques, et
- les agents amphotères,
et qui présente, à l'état dilué dans une solution aqueuse, prêt à l'emploi, une valeur pH d'au moins 10,5, permet d'éliminer/d'inactiver des micro-organismes choisis parmi le groupe constitué de bactéries, de virus et de champignons.

23. Procédé selon la revendication 22, **caractérisé en ce que** l'étape de nettoyage et/ou de désinfection a) constitue la première étape du procédé.

24. Procédé selon la revendication 22, **caractérisé en ce qu'**une étape de nettoyage a lieu avant l'étape de nettoyage et/ou de désinfection a).

25. Procédé selon la revendication 24, **caractérisé en ce qu'**on utilise lors de l'étape de nettoyage préalable le même détergent que lors de l'étape de nettoyage et/ou de désinfection a).

26. Procédé selon la revendication 25, **caractérisé en ce que** la concentration du détergent lors de l'étape de nettoyage préalable est inférieure à celle lors de l'étape de nettoyage et/ou de désinfection a) .

27. Procédé selon la revendication 26, **caractérisé en ce que** la concentration du détergent lors de l'étape de nettoyage préalable est inférieure au moins de moitié à celle lors de l'étape de nettoyage et/ou de désinfection a).

28. Procédé selon l'une quelconque des revendications 22 à 27, **caractérisé en ce qu'**il ne comprend aucune étape de désinfection thermique.
